Europäisches Patentamt

(19) European Patent Office

Office européen des brevets

(11) Publication number: **0 020 157**
**B1**

## (12) EUROPEAN PATENT SPECIFICATION

(45) Date of publication of patent specification: **05.10.83**  (51) Int. Cl.³: **A 61 L 11/00**

(21) Application number: **80301799.5**

(22) Date of filing: **30.05.80**

(54) Device for destroying bacterial flora.

(30) Priority: **01.06.79 AU 9032/79**

(43) Date of publication of application:
**10.12.80 Bulletin 80/25**

(45) Publication of the grant of the patent:
**05.10.83 Bulletin 83/40**

(84) Designated Contracting States:
**AT BE CH DE FR GB IT LI LU NL SE**

(56) References cited:
**DE - A - 2 503 605**
**US - A - 2 708 074**

(73) Proprietor: **Paull, Noel W.**
**8 Cheryl Grove**
**Viewbank Victoria (AU)**

(73) Proprietor: **Gemmell, Craig**
**72 Silverdale Road**
**Eaglemont Victoria (AU)**

(72) Inventor: **Gemmell, Leslie Wallace**
**72, Silverdale Road**
**Eaglemont Victoria (AU)**

(74) Representative: **Abbie, Andrew Kenneth et al,**
**A.A. THORNTON & CO. Northumberland House**
**303/306 High Holborn**
**London, WC1V 7LE (GB)**

Courier Press, Leamington Spa, England

## Device for destroying bacterial flora

This invention relates to a device for destroying bacterial flora by the sterilization of contaminated waste which contains the bacterial flora.

In hospitals, for example, waste materials are often disposed of by pouring the material down sinks and the like. This waste material may therefore gather in the S-bends or the like, of drainage pipes where bacterial flora in the waste material flourishes.

The bacterial flora which builds up on the drainage pipe is extremely dangerous, particularly in the hospital environment where it is possible that sterile equipment may be contaminated by the bacterial flora. Indeed, it has been known for a patient in a hospital to contract a sickness which has been attributed to bacterial flora contamination of instruments which have been used in connection with the patient. The present invention seeks to overcome this problem by providing a device which will destroy bacterial flora which gather in the drainage pipes.

The present invention includes a device for destroying bacterial flora by sterilization of contaminated waste which contains the bacterial flora, said device comprising an elongate housing having an inlet to allow waste material to pass into said housing and an outlet to allow waste material to pass out of the housing, said inlet and said outlet being couplable in a drainage pipeline such that said housing is disposed with its longitudinal axis extending transverse to the longitudinal axis of the portion of the drainage pipeline to which it is coupled, heating means in said housing for heating the waste material said heating means extending from one end of said elongate housing in the direction of the longitudinal axis of the housing such that any bacterial flora which gathers in or on said housing and drainage pipeline may be sterilized by heat from said heating means.

The present invention also includes a device for destroying bacteria by the sterilization of contaminated waste which contains the bacteria, said device comprising:

a housing having an inlet and an outlet for connecting in an S-bend of a drainage pipeline for allowing the waste material and water to pass into said housing for forming a plug in the housing and S-bend;

a heating means associated with said housing;

an actuating means for locating in the path of said water material in said drainage pipe such that when said waste material passes said actuating means, said actuating means actuates the heating means to boil the waste material and water which forms the plug in said housing and S-bend, the boiling action of the water and waste material extending throughout the housing and drainage pipe between a basin and outlet of the S-bend to destroy bacteria in the waste material and water as well as bacteria on the inside of the drainage pipeline.

We have been made aware of U.S. Specification No. 2,708,074 and German Offenlegungsschrift Nr. 25.03.605 both of which are concerned with destroying bacterial flora in hospital waste by sterilization. However, neither of these specifications discloses a device for destroying bacteria by the sterilization of contaminated waste which contains the bacteria comprising a housing provided with heating means and couplable directly into a drainage pipeline for sterilizing the contaminated waste as it passes through the discharge pipeline.

In order that the invention will be well understood two embodiments thereof, which are given by way of example only will now be described, reference being made to the accompanying drawings, in which:

Figure 1 is a perspective view of a device for destroying bacterial flora by sterilization of contaminated waste which contains the bacterial flora;

Figure 2 is a longitudinal, cross-sectional view of the device shown in Figure 1;

Figure 3 is a schematic diagram of a circuit for supplying power to the device shown in Figures 1 and 2;

Figure 4 is a sectional view of a second and preferred device for destroying bacterial flora by sterilization of contaminated waste containing the bacterial flora; and

Figure 5 is a circuit diagram for the device shown in Figure 4.

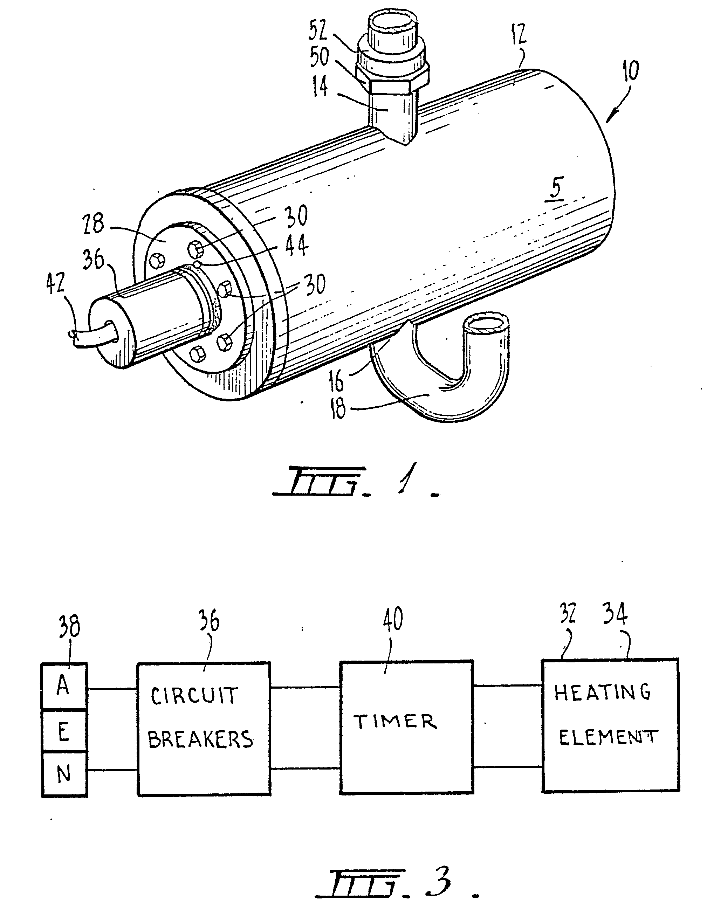

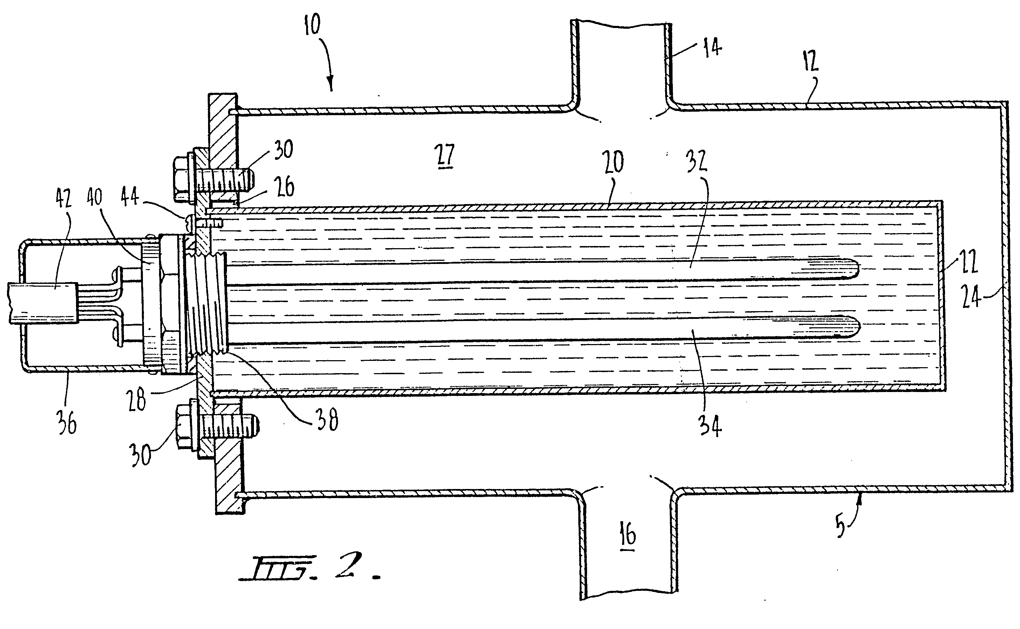

Referring to Figures 1 and 2, the device 10 comprises first outer member 12 of generally cylindrical form. The first member 12 has an inlet 14 and an outlet 16. The outlet 16 may be a straight pipe which is connected with a drainage pipeline (not shown) above the S-bend thereof or it may have an S-bend 18 integral therewith.

A second cylindrical member 20 is provided in the first member 12 and forms therewith an elongate housing 5 as best seen in Figure 2 which is couplable by means of inlet 14 and outlet 16 in the drainage pipeline, such that the housing 5 is disposed with its longitudinal axis extending transverse to the longitudinal axis of the portion of the drainage pipeline to which it is coupled. The second member 20 is spaced from the first member at their ends 22 and 24 and about the circumference of the members. The first and second members define a chamber 27 in which contaminated waste can gather as will be explained hereinafter.

The outer member 12 has an open end 26 which is closed by a plate 28 rigidly connected to the second cylindrical member 20 when the second cylindrical member 20 is located within the first member 12.

The plate 28 is connected to the first member 12 by bolts 30 to securely maintain the second member 20 within the first member 12.

A pair of heating elements 32 and 34 are removably located in the second member 20 extending from one end of the housing 5 in the direction of the longitudinal axis thereof. The elements 32 and 34 are held in a cap 36 which is screw threaded by thread 38 into an opening in the plate 28 which opening is provided with a cooperating screw thread. The elements 32 and 34 are supported by a support 40 and are independently powered by electrical energy supplied via an insulated conductor 42.

An air bleed 44 may be provided through plate 28 to allow escape of any air from the interior of the second member 20. The second member 20 is filled with a fluid having a high boiling point, for example, non-carbon oil.

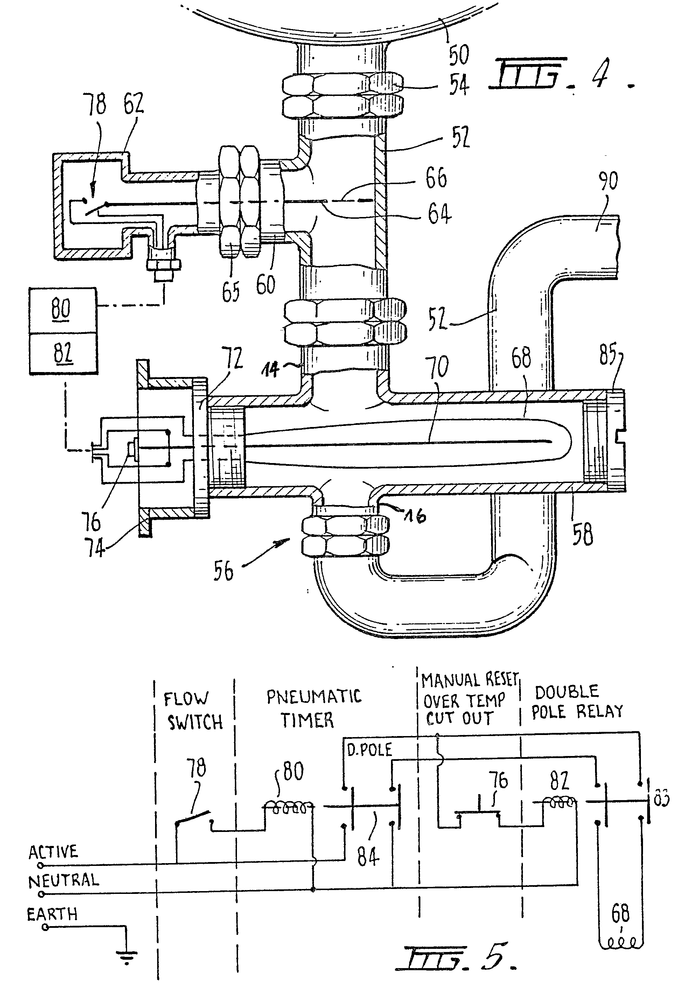

Figure 3 shows a schematic circuit diagram for supplying power to the heating elements 32 and 34. The electrical energy is first conveyed through circuit breakers 36 from a conventional 240 V outlet 38 to a timer 40 and then to the elements 32 and 34. The circuit breaker is used to disconnected power in the event of a failure in the heating elements 32 and 34 which may otherwise cause a "black out" in the area in which the device is used. The circuit breakers 36 are particularly advantageous if the device is to be used in a hospital to prevent any possibility of power supply disruption to essential equipment maintained in the hospital.

The timer 40 which may be a Venner synchronous motor-driven multiset time switch may be used to selectively supply power to the elements 32 and 34 as it may not be necessary to continually heat the oil in the second member 20. Indeed, if the sink or the like, beneath which the device is connected is not used regularly, it may also be necessary to supply energy to the elements for a few hours a day.

In operation, contaminated waste which is poured into the sink will pass down the drainage pipeline (not shown) and through the device 10. Generally, the contaminated waste will be flushed down the sink with water from a tap above the basin (not shown) to which the drainage pipeline and device 10 are attached. As the waste passes through the device it will tend to build up on the inside surface of the first member 12 and on the outside surface of the second member 20 as well as in the S-bend 18. Electrical energy which is supplied to the elements 32 and 34 will cause the same to heat the oil contained with the second member 20. Since oil has a high boiling point, the oil will become extremely hot and conduct heat to the second member 20. The second member 20 will in turn conduct heat into the first member 12 with the result that the entire device 12 will become extremely hot. The heat will therefore destroy any bacterial flora which may be present in the waste which gathers in the device 10 and any water and waste trapped in the housing 5 will be boiled, which boiling will also destroy bacteria in this waste and the boiling water will assist in destroying bacteria on the inside of the drainage pipeline. The extent of heat conduction in the inlet and outlet pipes 14 and 16 can be controlled by selecting an appropriate length of inlet and outlet pipe and securing the same to the drainage pipeline with appropriate connections (for example, connectors 50 and 52 in Figure 1) with a heat resistant gasket disposed therebetween.

Preferably, the first and second members 12 and 20 are fabricated from 16 gauge copper tube and the ends of these members which are joined are preferably silver braised.

In the second and preferred embodiment shown in Figures 4 and 5, a basin 50 has a drainage pipeline 52 connected therewith by a coupling 54. Formed in the S-bend of 56 of the pipeline 52 is an elongate housing forming a water cylinder 58. The cylinder 58 may be formed integral with the pipeline 52 or may be welded or otherwise coupled into the pipeline. As can be seen from Figure 4 the cylinder has an inlet 14 and an outlet 16 which are coupled to the drainage pipeline such that the cylinder is disposed with its longitudinal axis extending transverse to the longitudinal axis of the portion of drainage pipeline to which it is coupled. Above the cylinder 58, a branch 60 is provided and a flow switch 62, preferably a FS15 flow switch manufactured by M. E. Mack & Co. Pty. Ltd., of Box Hill, Victoria, Australia, is connected to the branch 60 by a coupling 65. The flow switch 62 includes a tongue or paddle 64 which extends into pipeline 52 substantially across the diameter of the pipeline 52. The tongue 64 has an aperture 66 therein to prevent waste material flushed down the sink from being trapped above the tongue 64.

The branch 60 and flow switch 62 form an angle $\alpha$ with the pipeline 52 of preferably about 85° so that the branch is slightly inclined to prevent water from building up or remaining in the branch 60. The cylinder 58 includes a heating element 68 and a probe 70 of an over-temperature cut-out mechanism to be described hereinafter.

The heating element 68 and probe 70 extend from one end of the cylinder 58 in the direction of the longitudinal axis thereof. More specifically, heating element 68 and probe 78 are mounted on a member 72 which is screw-threaded into the cylinder 58 and contains contacts for supplying power to the element 68. A cover 74 is provided at the end of the cylinder 58 to house the member 72 and a manual reset cut-out switch 76.

The probe 70 has a bellows type heat sensor or thermostat, preferably that made by T. L. Thermatic Controls Ltd., Canada, certified under E53438 therein, which is coupled to the switch 76 so that when the temperature in the cylinder 58 reaches a predetermined value the bellows expands to open switch 76 which normally closed.

The function of the flow switch 62 is well known and will therefore not be fully described. Suffice it to say that when the tongue 64 is contacted by water of waste material, the tongue is displaced downwardly in Figure 4 to actuate a micro switch represented schematically at 78 to complete a circuit to a pneumatic timer 80. The timer 80 is preferably an Agastat (trade mark) 7000 Series timer made by Amerace Ltd. of Ontario, Canada. The timer may be "preprogrammed" to supply power, once actuated for a predetermined amount of time, to the heating element 68 in combination with a double pole relay 82 preferably manufactured by N. H. P. Pty. Ltd., of Rivers Street, Richmond, Victoria, Australia, the function of which shall be clear from the following description of Figure 5.

Referring to Figure 5 the micro-switch contact 78 which is normally open is closed by movement of tongue 64 when water or waste material is flushed down pipeline 52. Closure of switch 78 supplies power to timer 80 which closes a double pole switch 84 which in turn supplies power to the double pole relay 82 which causes double pole switch 83 to close and thereby supply power to heating element 68.

The over temperature cut-out switch 76 is provided in the series circuit to the double pole relay 82 and is coupled to the bellows (not shown) located in probe 70.

If the temperature of the probe 70 reaches a predetermined value, for example a temperature indicative of the cylinder 58 boiling dry, the bellows (not shown) expands and causes switch 76 to open which in turn cuts off power to relay 82 which opens switch 83 to cut off power to element 68 to prevent the element from burning out. The switch 76 must be reset manually in order to again supply power to relay 82.

Since the cylinder 58 is located in S-bend 56 of the pipeline 52, the cylinder will normally have water therein, which water forms the normal plug in the S-bend. Upon water and waste material being passed down pipeline 52, the flow switch 62 will actuate the heating element 68 as detailed above, to cause the water and waste material in cylinder 58 and the remainder of the S-bend 56 to be heated.

As noted above, the timer 80 may be programmed to supply power for a predetermined amount of time. It has been found that about 6 minutes is required to boil the water in the S-bend 56 and depending upon the nature of the bacteria which accumulates in the pipe 52, a further 2, to about 24 minutes may be required to destroy the bacteria in the pipe.

Accordingly, the timer 80 may be programmed to supply power to the element 68 for between 0 and 30 minutes to destroy bacteria, with about 8 to 10 minutes being found the most suitable time period. Once this period has elapsed the timer automatically shuts off power to the element and remains in this condition until actuated by the flow switch 62.

When actuated, the element 68 which is in contact with the water and waste material in the S-bend 56, boils the same to destroy bacteria therein. The boiling action in the cylinder causes boiling water to bubble throughout the pipeline 52 and heat from the boiling water is circulated through the pipeline 52, branch 60 and cylinder 58 to destroy any bacteria which may adhere to the surfaces of these members.

Any bacteria, which of course passes out of pipeline 52 is not a problem since it is not capable of contaminating sterile equipment.

Heat resistant washers (not shown) may also be provided in the coupling 54 and at the outlet 90 of the pipeline 52 to prevent heat conduction beyond pipe 52. A steam trap (not shown) may also be provided at the top of the pipeline 52 to prevent steam from escaping from sink 50.

The cylinder 58 may have a screw threaded inspection plug 85 which may be unscrewed to allow the inside of the cylinder to be inspected.

The illustrated embodiments destroy substantially all bacteria which otherwise has been found to flourish in contaminated waste and the like which adheres to drainage pipes.

**Claims**

1. A device for destroying bacterial flora by sterilization of contaminated waste which contains the bacterial flora, said device comprising an elongate housing (5, 58) having an inlet (14) to allow waste material to pass into said housing and an outlet (16) to allow waste material to pass out of the housing (5, 58) said inlet (14) and said outlet (16) being couplable in a drainage pipeline (56) such that said housing is disposed with its longitudinal axis extending transverse to the longitudinal axis of the portion of the drainage pipeline to which it is coupled, heating means (32, 34, 68) in said housing for heating the waste material said heating means (32, 34, 68) extending from one end of said elongate housing (5, 58) in the direction of the longitudinal axis of the housing (5, 58) such that any bacterial flora which gathers in or on said housing (5, 58) and drainage pipeline (56) may be sterilized by heat from said heating means.

2. A device according to claim 1, wherein said housing (5) is defined by a first member (12) which receives a second member (20) therein, the second member (20) being in contact with said first member (12) at least along one side and said second member containing said heating means (32, 34) such that heat from the heating means may be conducted from the second member (20) to the first member (12), said heating means comprising two heating elements (32, 34) releasably sealed within said second member (20) and said second member (20) containing fluid for conducting heat from the elements (32, 34) to the second member (20) such that waste which gathers in said housing (5) between the first and second members may be boiled by heat conducted from the second member to destroy bacteria in the housing.

3. A device according to claim 1 wherein said housing comprises a water collecting housing (58) said water collecting housing (58) having the heating means (68) therein such that waste material collected in said housing may be boiled by said heating means (68) to destroy bacteria therein.

4. A device according to claim 1, 2 or 3, wherein said housing (5, 58) is located in an S-bend (56) in the drainage pipeline (52) such that waste material and water which generally form a water plug in the S-bend (56) will also be trapped in the housing (5, 58).

5. A device according to any one of the preceding claims wherein said device includes an actuator (62) for actuating said heating means (68) when water or waste material passes into the housing (58).

6. A device according to claim 5, wherein said actuator (62) comprises a tongue member (64) projecting into the path of waste material and water into the housing (58) such that the water or waste material contacting said tongue (64) will cause the tongue (64) to move to close a contact (78) to thereby actuate the heating means (68).

7. The device according to claim 7, wherein a timing means (80) is coupled between said actuator (62) and said heating means (68) to actuate the heating means (68) for a desired time upon actuation of the actuator (62).

8. A device for destroying bacteria by the sterilization of contaminated waste which contains the bacteria, said device comprising:

a housing (58) having an inlet and an outlet for connecting in an S-bend (56) of a drainage pipeline (52) for allowing the waste material and water to pass into said housing for forming a plug in the housing and S-bend (56);

a heating means (68) associated with said housing (58);

an actuating means (62) for locating in the path of said water material in said drainage pipeline (52) such that when said waste material passes said actuating means (62), said actuating means actuates the heating means (68) to boil the waste material and water which forms the plug in said housing (58) and S-bend (56), the boiling action of the water and waste material extending throughout the housing (58) and drainage pipeline (52) between a basin (50) and outlet (90) of the S-bend (56) to destroy bacteria in the waste material and water as well as bacteria on the inside of the drainage pipeline (52).

9. A device according to claim 8, wherein said actuating means (62) is housed in a branch (60) connected to said drainage pipeline (52) above said housing (58), said branch (60) being inclined relative to said drainage pipeline (52) to prevent water and waste material from collecting in the branch (60), said actuator (62) including a tongue member (64) which extends into the drainage pipeline and a switch (78) such that when water and waste material contact the tongue member (64), the tongue member (64) is displaced to actuate the switch (78) to supply power to the heating means (68).

10. A device according to claim 9, wherein said tongue member (64) is provided with at least one aperture (66) to allow waste material in solid form to pass through the aperture.

11. A device according to claim 8, 9 or 10, wherein a timer means (80) is coupled between the actuating means (62) and the heating means (68) such that upon actuation of the actuating means (62) the timer means (80) supplies power to the heating means (68) for a desired amount of time to cause boiling of the water and waste material.

12. A device according to any one of claims 8 to 11, wherein said heating means comprises a heating element (68) disposed in said housing for directly heating the water and waste material in said housing.

13. A device according to any one of claims 8 to 12, including shut-off means (70, 76) for shutting off said heating element (68) if the water and waste material in the housing is completely boiled off or drops below a predetermined level.

**Patentansprüche**

1. Vorrichtung zum Zerstören von baktierieller Flora durch Sterilisation von die bakterielle Flora enthaltenden, kontaminierten Abfällen, umfassend ein längliches Gehäuse (5, 58) mit einem Einlaß (14), durch welchen hindurch Abfallmaterial in das Gehäuse gelangen kann, und einem Auslaß (16), durch welchen hindurch Abfallmaterial aus dem Gehäuse (5, 58) herausgelangen kann, wobei der Einlaß (14) und der Einlaß (16) derart in einer Abflußleitung (56) anschließbar sind, daß sich die Längsachse des Gehäuses quer zur Längsachse des Abschnitts der Abflußleitung erstreckt, an welchem es angeschlossen ist, ferner im Gehäuse angeordnete Heizeinrichtungen (32, 34, 68) zum Erhitzen des Abfallmaterials, wobei sich die Heizeinrichtungen (32, 34, 68) von einem Ende des länglichen Gehäuses (5, 58) aus in Richtung der Längsachse des Gehäuses (5, 58) erstrecken, so daß jegliche sich in oder an dem Gehäuse (5, 58) und der Abflußleitung (56) ansammelnde bakterielle Flora durch die von den Heizeinrichtungen erzeugte Hitze sterilisiert werden kann.

2. Vorrichtung nach Anspruch 1, bei welcher das Gehäuse (5) von einem ersten Teil (12) umgrenzt ist, welches ein zweites Teil (20) aufnimmt, so daß das zweite Teil (20) wenigstens entlang einer Seite mit dem ersten Teil (12) in Berührung steht und die Heizeinrichtungen (32, 34) enthält, wodurch von den Heizeinrichtungen erzeugte Hitze vom zweiten Teil (20) auf das erste Teil (12) übergeleitet werden kann, wobei die Heizeinrichtungen zwei entnehmbar und abdichtend im zweiten Teil (20) angeordnete Heizelemente (32, 34) aufweisen und das zweite Teil (20) ein Strömungsmittel für die Überleitung von Wärme von den Elementen (32, 34) auf das zweite Teil (20) enthält, so daß sich in dem Gehäuse (5) zwischen dem ersten und dem zweiten Teil ansammelnde Abfälle durch vom zweiten Teil abgeleitete Wärme gekocht werden können, um Bakterien in dem Gehäuse zu zerstören.

3. Vorrichtung nach Anspruch 1, bei welchem das Gehäuse ein Wassersammelgehäuse (58) aufweist, in welchem die Heizeinrichtungen (68) angeordnet sind, so daß in dem Gehäuse gesammeltes Abfallmaterial durch die Heizeinrichtungen (68) gekocht werden kann, um darin enthaltene Bakterien zu zerstören.

4. Vorrichtung nach Anspruch 1, 2 oder 3, bei welchem das Gehäuse (5, 58) in einem S-Krümmer (56) einer Abflußleitung (52) angeordnet ist, so daß Abfallmaterial und Wasser, welches gewöhnlich einen Wasserverschluß im S-Krümmer (56) bildet, ebenfalls in dem Gehäuse (5, 58) eingeschlossen wird.

5. Vorrichtung nach einem der vorstehenden Ansprüche, bei welcher die Vorrichtung eine Betätigungseinrichtung (62) aufweist, mittels welcher die Heizeinrichtungen (68) beim Eintritt von Wasser oder Abfallmaterial in das Gehäuse (58) betätigbar sind.

6. Vorrichtung nach Anspruch 5, bei welcher die Betätigungseinrichtung (62) ein in den in das Gehäuse (58) hinein führenden Weg des Abfallmaterials und Wassers ragendes Zungenglied (64) aufweist, welches durch Beaufschlagung mit Wasser oder Abfallmaterial zum Schließen eines Kontakts (78) für die Betätigung der Heizeinrichtungen (68) bewegbar ist.

7. Vorrichtung nach Anspruch 6, bei welchem zwischen der Betätigungseinrichtung (62) und den Heizeinrichtungen (68) eine Zeitgebereinrichtung (80) angeordnet ist, über welche die Heizeinrichtungen (68) bei Betätigung der Betätigungseinrichtung (62) über eine gewünschte Zeitspanne betätigbar sind.

8. Vorrichtung zum Zerstören von Bakterien durch Sterilisierung von die Bakterien enthaltendem, kontaminiertem Abfall, umfassend

ein Gehäuse (58) mit einem Einlaß und einem Auslaß für den Anschluß in einem S-Krümmer (56) einer Abflußleitung (52), so daß Abfallmaterial und Wasser in das Gehäuse eindringen können, um einen Verschluß im Gehäuse und im S-Krümmer (56) zu bilden;

eine dem Gehäuse (58) zugeordnete Heizeinrichtung (68);

eine Betätigungseinrichtung (62) für die Anordnung in dem Weg des Wassers und Materials in der Abflußleitung (52) derart, daß wenn das Abfallmaterial die Betätigungseinrichtung (62) passiert die Betätigungseinrichtung die Heizeinrichtung (68) betätigt, um das den Verschluß im Gehäuse (58) und im S-Krümmer (56) bildende Abfallmaterial und Wasser zu kochen, wobei sich die Kochwirkung des Wassers und des Abfallmaterials zwischen einem Becken (50) und einem Auslaß (90) des S-Krümmers (56) durch das gesamte Gehäuse (58) und die Abflußleitung (52) erstreckt, um im Abfallmaterial und im Wasser enthaltene Bakterien sowie Bakterien an der Innenseite der Abflußleitung (52) zu zerstören.

9. Vorrichtung nach Anspruch 8, bei welcher die Betätigungseinrichtung (62) in einer oberhalb des Gehäuses (58) mit der Abflußleitung (52) verbundenen Zweigleitung (60) untergebracht ist, wobei, die Zweigleitung (60) relativ zur Abflußleitung (52) geneigt ist, um die Ansammlung von Wasser und Abfallmaterial in der Zweigleitung (60) zu verhindern, und die Betätigungseinrichtung (62) ein in die Abflußleitung hineinragendes Zungenglied (64) sowie einen Schalter (78) aufweist, so daß das Zungenglied (64) bei Beaufschlagung von Wasser und Abfallmaterial bewegt wird, um den Schalter (78) für die Speisung der Heizeinrichtung (68) mit Energie zu betätigen.

10. Vorrichtung nach Anspruch 9, bei welcher das Zungenglied (64) mit wenigstens einer Öffnung (66) versehen ist, durch welche Abfallmaterial in fester Form hindurchtreten kann.

11. Vorrichtung nach Anspruch 8, 9 oder 10, bei welchem zwischen der Betätigungseinrichtung (62) und der Heizeinrichtung (68) eine Zeitgebereinrichtung (80) derart angeordnet ist, daß die Zeitgebereinrichtung (80) bei Betätigung der Betätigungseinrichtung (62) die Heizeinrichtung (68) während einer gewünschten Zeitspanne mit Energie speist, um das Kochen des Wassers und Abfallmaterials zu bewirken.

12. Vorrichtung nach einem der Ansprüche 8 bis 11, bei welcher die Heizeinrichtung ein Heizelement (68) aufweist, welches in dem Gehäuse angeordnet ist, um das Wasser und Abfallmaterial in dem Gehäuse direkt zu erhitzen.

13. Vorrichtung nach einem der Ansprüche 8 bis 12, umfassend eine Abschalteinrichtung (70, 76) zum Abschalten des Heizelements (68) im Falle daß das Wasser und Abfallmaterial im Gehäuse vollkommen weggekocht ist oder unter eine vorbestimmte Standhöhe abfällt.

## Revendications

1. Dispositif permettant de détruire la flore bactérienne par stérilisation des matières usées contaminées contenant cette flore bactérienne, ledit dispositif comprenant un récipient allongé (5, 58) possédant une entrée (14) permettant aux matières usées de pénétrer dans ledit récipient et une sortie (16) permettant aux matières usées de sortir du récipient (5, 58), ladite entrée (14) et ladite sortie (16) pouvant être raccordées à un tuyau d'évacuation (56) de telle façon que ledit récipient ait son axe longitudinal disposé transversalement à l'axe longitudinal de la partie du tuyau d'évacuation à laquelle il est raccordé, un moyen de chauffage (32, 34, 68) disposé dans ledit récipient pour chauffer les matières usées, ledit moyen de chauffage (32, 34, 68) partant d'une première extrémité dudit récipient allongé (5, 58) et se prolongeant suivant l'axe longitudinal du récipient (5, 58), si bien que toute la flore bactérienne qui s'est rassemblée dans ou sur le récipient (5, 58) et le tuyau d'évacuation (56) peut être stérilisée par la chaleur émanant dudit moyen de chauffage.

2. Dispositif selon la revendication 1, où ledit récipient (5) est défini par un premier élément (12) qui loge un deuxième élément (20), le deuxième élément (20) étant en contact avec le premier élément (12) suivant au moins un côté et ledit deuxième élément contenant ledit moyen de chauffage (32, 34) de sorte que la chaleur émanant du moyen de chauffage peut être conduite du deuxième élément (20) au premier élément (12), ledit moyen de chauffage comprenant deux éléments chauffants (32, 34) logés de manière étanche et amovible à l'intérieur dudit deuxième élément et ledit deuxième élément (20) contenant un fluide permettant de conduire la chaleur des éléments (32, 34) au deuxième élément (20) de façon que les matières usées qui se sont rassemblées dans ledit récipient (5) entre le premier élément et le deuxième élément puissent être mises en ébullition par la chaleur conduite à partir du deuxième élément en vue de la destruction des bactéries contenues dans le récipient.

3. Dispositif selon la revendication 1, où ledit récipient comprend un récipient (58) de recueil d'eau, ledit récipient de recueil d'eau (58) contenant le moyen de chauffage (68) de façon que les matières usées recueillies dans ledit récipient puissent être mises en ébullition par ledit moyen de chauffage (68) en vue de la destruction des bactéries qui s'y trouvent.

4. Dispositif selon la revendication 1, 2 ou 3, où ledit récipient (5, 58) est placé dans un syphon (56) du tuyau d'évacuation (52) de façon que les matières usées et l'eau qui forment généralement un bouchon d'eau dans le syphon (56) soient également retenues dans le récipient (5, 58).

5. Dispositif selon l'une quelconque des précédentes revendications, où ledit dispositif comporte un moyen d'actionnement (62) permettant de mettre en service ledit moyen de chauffage (68) lorsque l'eau ou les matières usées pénètrent dans le récipient (58).

6. Dispositif selon la revendication 5, où ledit moyen d'actionnement (62) comprend une languette (64) faisant saillie sur le trajet des matières usées et de l'eau dans le récipient (58) de sorte que l'eau ou les matières usées entrant en contact avec ladite languette (64) amènent la languette (64) à se déplacer pour fermer un contact (78) de façon à mettre en service le moyen de chauffage (68).

7. Dispositif selon la revendication 6, où une minuterie (80) est connectée entre ledit moyen d'actionnement (62) et ledit moyen de chauffage (68) de façon à mettre en service le moyen de chauffage (68) pendant une durée voulue après l'actionnement du moyen d'actionnement (62).

8. Dispositif permettant de détruire les bactéries par la stérilisation de matières usées contaminées contenant les bactéries, ledit dispositif comprenant:

un logement (58) possédant une entrée et une sortie permettant son raccordement sur le syphon (56) d'un tuyau d'évacuation (52) de façon à permettre aux matières usées et à l'eau de pénétrer dans ledit récipient pour former un bouchon dans le récipient et le syphon (56);

un moyen de chauffage (68) associé audit récipient (58);

un moyen d'actionnement (62) destiné à être placé sur le trajet desdites matières usées et de l'eau dans ledit tuyau d'évacuation (52) de façon que, lorsque, lesdites matières usées passent sur ledit moyen d'actionnement (62), ce dernier met en service le moyen de chauffage (68) pour faire bouillir les matières usées et l'eau qui forment le bouchon dans ledit récipient (58) et le syphon (56), l'ébullition de l'eau et des matières usées s'étendant dans tout le récipient (58) et le tuyau d'évacuation (52) entre une cuvette (50) et une sortie (90) du syphon (56) de façon à détruire les bactéries contenues dans les matières usées et l'eau aussi bien que les bactéries se trouvant sur l'intérieur du tuyau d'évacuation (52).

9. Dispositif selon la revendication 8, où ledit moyen d'actionnement (62) est logé dans une dérivation (60) raccordée audit tuyau d'évacuation (52) au-dessus dudit récipient (58), ladite dérivation (60) étant inclinée par rapport audit tuyau d'évacuation (52) de façon à empêcher que l'eau et les matières usées ne soient recueillies dans la dérivation (60), ledit moyen d'actionnement (62) comportant une languette (64) qui se prolonge jusque dans le tuyau d'évacuation et un interrupteur (78) si bien que, lorsque l'eau et les matières usées entrent en contact avec la languette (64), cette dernière se déplace et actionne l'interrupteur (78) de façon à délivrer du courant électrique au moyen de chauffage (68).

10. Dispositif selon la revendication 9, où ladite languette (64) comporte au moins une ouverture (66) de façon que les matières usées sous forme solide puissent passer au travers de l'ouverture.

11. Dispositif selon la revendication 8, 9 ou 10, où une minuterie (80) est connectée entre le moyen d'actionnement (62) et le moyen de chauffage (68) si bien que, après actionnement du moyen d'actionnement (62), la minuterie (80) délivre courant électrique au moyen de chauffage (68) pendant une durée voulue pour provoquer l'ébullition de l'eau et des matières usées.

12. Dispositif selon l'une quelconque des revendications 8 à 11, où ledit moyen de chauffage comprend un élément chauffant (68) disposé dans ledit récipient de façon à chauffer directement l'eau et les matières usées se trouvant dans ledit récipient.

13. Dispositif selon l'une quelconque des revendications 8 à 12, comportant un moyen de coupure (70, 76) permettant de couper l'alimentation dudit élément chauffant (68) si l'eau et les matières usées se trouvant dans le récipient s'évaporent complètement par ébullition ou tombent au-dessous d'un niveau prédéterminé.

FIG. 1.

| A E N | CIRCUIT BREAKERS | TIMER | HEATING ELEMENT |

FIG. 3.

FIG. 2.

0 020 157

0 020 157

FIG. 4.

FIG. 5.